# EUROPEAN PATENT SPECIFICATION

(11) **EP 2 085 104 B1**
(45) Date of publication and mention of the grant of the patent: **01.04.2015**
(21) Application number: 06840738.6
(22) Date of filing: 30.12.2006
(51) Int. Cl.: A61M 5/315, A61M 5/50, A61M 5/32

(54) **MEDICAL SAFETY INJECTOR AND PLUNGER COMBINATION FOR MEDICAL SAFETY INJECTION**
MEDIZINISCHES SICHERHEITSINJEKTIONSGERÄT UND KOLBENKOMBINATION FÜR MEDIZINISCHE SICHERHEITSINJEKTION
INJECTEUR MÉDICAL SÉCURISÉ ET ENSEMBLE DE PISTON POUR LA RÉALISATION D'UNE INJECTION MÉDICALE SÉCURISÉE

(30) Priority: 28.09.2006 CN 200610141063
(43) Date of publication of application: 05.08.2009
(73) Proprietor: Bencha International Group Inc., Road Town Tortola (VG)
(72) Inventor: LIN LEE, Lee, Taipei City, Taiwan 104 (CN)
(74) Representative: Lang, Christian
(86) International application number: PCT/CN2006/003708
(87) International publication number: WO 2008/037138

(56) References cited:
- EP-A1- 1 192 967
- CN-Y- 2 678 668
- US-A- 5 053 010
- US-A- 6 086 568
- US-B2- 6 752 782

## Description

### BACKGROUND OF THE INVENTION

### 1. Technical Field

The present invention relates to a safety injector, and more particularly, to an automatically retractable medical safety injector and a plunger combination thereof that can be operated with a single hand and allow a needle to retract thereinto after use.

### 2. Description of Related Art

In order to reduce the risk of cross infection of such diseases as AIDS, hepatitis B or hepatitis C through blood during conventional medical injection, prohibit repeated use of a same injector by drug addicts and protect medical care workers from inadvertent injury by used needles during medical injection, syringes widely used nowadays generally adopt a non-reusable safety design, i.e., they are implemented as disposable syringes to be discarded after a single use. However, to facilitate application of force by a user performing an injection, most of such disposable safety syringes are designed to be held by both hands at a fixed angle during injection. Consequently, if a diabetic who has to inject insulin by himself cannot operate a syringe with both hands, the safety syringe has to be replaced by a common one, thereby increasing the risk of contracting dangerous infectious diseases.

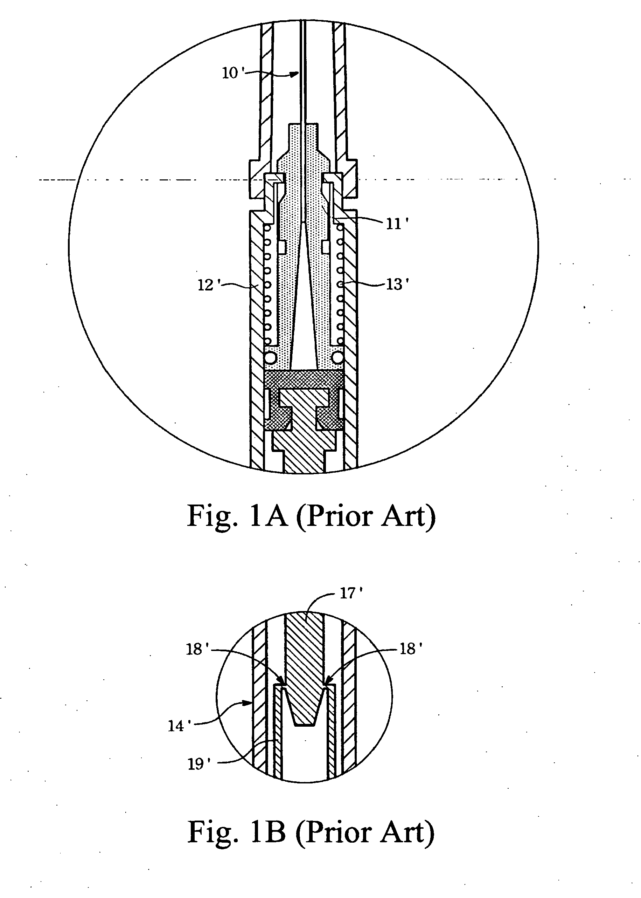

To solve this problem, a novel safety syringe that facilitates single-handed operation is proposed in Taiwan Patent No. 520995, entitled "Automatically Retractable Safety Syringe", and U.S. Patent No. 6,712,793 B1, entitled "Needle Guard Assembly for the Needle of a Syringe Body" which discloses all the features of the preamble of claim 1. As shown in Figs. 1A and 1B, this syringe comprises a hollow barrel 12', a needle hub 11' engaged with a needle

10', a helical retractile spring 13 and a breakable retracting plunger 14'. The breakable retracting plunger 14' comprises a front plunger section 17', a breakable connection 18' and a hollow rear plunger section 19' formed integrally as one piece. Once the breakable retracting plunger 14' is assembled into the hollow barrel 12' to complete a medical injection, further application of force toward the needle 10' will break the breakable connection 18', causing the front plunger section 17' to retract into a hollow portion of the hollow rear plunger section 19'. Thus, space in the hollow barrel 12'is freed for receiving the needle hub 11' along with the needle 10'.

However, to facilitate force application and operation by an operator, the breakable connection of the breakable retracting plunger must be made with high symmetry and high precision, which requires very tight dimensional tolerance of molds used to injection mold the plunger in one piece. In addition, it is very difficult to prevent the plunger from being broken during assembly and handling. As a result, the pass rate and production output cannot be effectively increased while the cost of the mold remains high. Furthermore, the precisely designed breakable connection 18' must work with a particular force application angle in order for the plunger to retract for safety. Hence, in case the injection site has a large curvature or is on a delicate part of the human body, such as the head, the back of the ears, the eyes, the oral cavity or the like, not only is it difficult for a medical care worker to perform the injection with a single hand, but it is also very likely that the function of the syringe will be inadvertently compromised due to a restricted injection angle and operational inconvenience, causing discomfort to the patient.

US 6,086,568 describes a syringe plunger rod for retracting needle syringe wherein the retracted needle syringe is received in an inner and an outer member of the plunger rod.

### BRIEF SUMMARY OF THE INVENTION

A primary objective of this invention is to provide a medical safety injector and a plunger combination thereof that can be conveniently operated with a single hand and are less likely to be damaged due to an improper operation angle or posture of an operator during an injection process.

Another objective of this invention is to provide a medical safety injector and a plunger combination thereof that are less likely to be damaged during assembly so as to have higher quality, pass rate and reliability while waste of raw materials and processing costs are reduced.

A further objective of this invention is to provide a medical safety injector and a plunger combination thereof that can be easily made by plastic injection molding with a relaxed requirement of mold precision, so that the allowable error rate of product quality falls within a reasonable range to lower production costs.

Yet another objective of this invention is to provide a medical safety injector and a plunger combination thereof that are capable of buffering force applied thereto and resisting lateral shear so as to improve operational convenience, decrease injection failure rate and relieve patients of suffering.

Yet a further objective of this invention is to provide a medical safety injector and a plunger combination thereof that help increase the production capacity of molds in a single batch so as to raise production output remarkably.

Still another objective of this invention is to provide a medical safety injector and a plunger combination thereof wherein a plunger has a predetermined mechanical strength and satisfactory operability, so that a needle can automatically retract after use.

Based on the aforesaid and other objectives, this invention as defined by claim 1 provides an automatically retractable medical safety injector and a plunger combination contained therein for intramuscular injection, hypodermic injection, intravenous injection or drawing blood, wherein the injector and the plunger combination allow for only a single use.

The medical safety injector comprises: a needle hub connected with a needle; a hollow barrel for retaining the needle hub and, upon completion of injection, guiding the needle hub to retract in a direction opposite to an injection direction; and a plunger combination disposed within the hollow barrel, wherein the plunger combination comprises a retractable plunger and a hollow shank muff-coupled, and thus forming a muff-coupled region, with the retractable plunger. The retractable plunger an exterior sidewall formed with at least one protrusion in the muff-coupled region for being correspondingly snap-fitted with at least one recess formed on an interior sidewall of the hollow shank. If a user continues to apply a force to the plunger combination after the medical safety injector accomplishes an injection, the retractable plunger will retract into the hollow shank to free space in the hollow barrel for receiving the used needle hub.

In the medical safety injector disclosed in this invention, a relative position of the hollow shank and the retractable plunger of the plunger combination in a fore-and-aft direction can be reversed as desired. Particularly, to obtain a better effect in retracting the retractable plunger, according to this invention, one or more lateral stress-adjustment cutouts are provided on the retractable plunger at a core portion of the muff-coupled region where the retractable plunger is muff-coupled with the hollow shank, or at least one stress-adjustment cutout is further provided at an edge of a bevel surface of the hollow shank. The at least one stress-adjustment cutout formed at the edge of the bevel surface of the hollow shank keeps away longitudinally from the recess on the interior sidewall of the hollow shank.

To cope with various possible strengths or angles with or at which a force is applied single-handedly during an injection process, a further reinforcing design is provided for the retractable plunger and the hollow shank of the plunger combination in the medical safety injector of this invention.

According to a second embodiment disclosed hereunder, in the medical safety injector and plunger combination thereof of this invention, one or more vent holes are additionally provided in a sidewall of the hollow shank distal from the needle, so that appropriate ventilation is achieved through the vent holes on the hollow shank when the retractable plunger is retracting into the hollow shank, thereby preventing a user from feeling air resistance on his/her thumb and thus facilitating the user's operation. Similarly, if the retractable plunger of the plunger combination is proximate to a back end of the hollow barrel, one or more vent holes may also be additionally provided in the sidewall of the retractable plunger distal from the needle, thereby achieving good ventilation through the vent holes.

According to a third and a fourth embodiment disclosed hereunder, the aforesaid protrusion on the exterior sidewall of the retractable plunger is replaced by at least one lateral annular protruding rib or one or more rows of lateral protruding spots adjacent to or spaced apart from each other. Meanwhile, on the interior sidewall of the hollow shank, at least one annular concave rib or one or more rows of annular grooves adjacent to or spaced apart from each other are correspondingly formed to replace the aforesaid recess.

Further, according to a fifth embodiment disclosed hereunder, in the muff-coupled region where the hollow shank is muff-coupled with the retractable plunger, a plurality of stoppers are formed on a sidewall of the hollow shank near a bottom edge of the retractable plunger along a retracting direction of the retractable plunger, or alternatively, a roughened portion is formed above the protrusion of the retractable plunger (as in the sixth embodiment), so as to counteract or buffer an excessively strong force applied by the user when pushing the plunger. This will prevent the retractable plunger from retracting into the hollow shank before an injection is completed and thereby avoid injection failure.

In order to improve production pass rate and operability of the medical safety injector and the plunger combination thereof of this invention, the needle hub also has an improved design. According to a seventh embodiment disclosed hereunder, in order to improve the pass rate of retracting operation of the needle hub, a narrow barrel portion having a diameter smaller than a diameter of the hollow barrel is formed integrally in a front section of the hollow barrel to receive a compressed spring and a portion of the needle hub. At an opening of the hollow barrel where the hollow barrel joins the narrow barrel portion, at least one hollow ring stopper (movable ring stopper) having a hollow portion whose inner diameter is smaller than a diameter of the portion of the needle hub is provided to prevent the needle hub from slipping out too far under an injection force and to guide the needle hub to retract upon completion of injection.

Besides the additional hollow stoppers, according to an eighth embodiment of this invention disclosed hereunder, an injection molding technique is used to form a bottleneck portion integrally at the opening where the hollow barrel joins the narrow barrel portion while the hollow barrel is formed, wherein the bottleneck portion only allows the needle and a portion of the needle hub to pass through. With the bottleneck portion having a central through-hole, it is also possible to stop the needle hub and maintain it at the best retracting position by controlling an extension distance of the needle hub.

Unlike the integrally formed breakable connection of the prior art plunger, the automatically retractable medical safety injector and the plunger combination thereof of this invention have a split configuration, in which the retractable plunger and the hollow shank are injection molded separately from a flexible material (e.g., plastic or rubber) and then assembled together. This may lower the risk of inadvertent damage to the plunger due to impact during assembly, and reduce injection faults attributed to damage of the plunger prior to unpacking and during use. Moreover, as the two pieces of the plunger are molded separately and then put together, the plunger may have a relaxed requirement of mold precision because it is no more necessary to consider force equilibrium at the connecting joint as in the prior art plunger integrally formed with the breakable connection. Hence, the development cost of molds is reduced while reliability and quality stability are improved.

On the other hand, the two-piece plunger combination exhibits better force equilibrium features than plungers with breakable connections of the existing syringes in terms of lateral shear strength. Therefore, when a patient performs an injection with a single hand or when a medical care worker performs an injection at a site difficult to access, the injection operation will not be affected by restrictions in the injection angle or posture.

### BRIEF DESCRIPTION OF THE SEVERAL VIEWS OF THE DRAWINGS

The invention as well as a preferred mode of use and advantages thereof will be best understood by referring to the following detailed description of illustrative embodiments in conjunction with the accompanying drawings, wherein:
Figs. 1A and 1B are cross-sectional views of a conventional automatically retractable safety injector having a plunger with a breakable connection;
Fig. 2 is an exploded perspective view of a medical safety injector and a plunger combination thereof according to a first embodiment of this invention;
Fig. 3 shows a cross-sectional view and a partial enlarged view of a plunger combination in the medical safety injector according to the first embodiment of this invention;
Fig. 4 is a schematic view illustrating how a retractable plunger and a hollow shank of the plunger combination are muff-coupled in the medical safety injector according to the first embodiment of this invention;
Fig. 5 is a partial perspective view of the hollow shank in the medical safety injector according to the first embodiment of this invention;
Figs. 6A and 6B are cross-sectional views of the medical safety injector in another implementation of the first embodiment of this invention;
Fig. 7 is an exploded cross-sectional view of a hollow shank in a medical safety injector according to a second embodiment of this invention;
Fig. 8 is a cross-sectional perspective view of a plunger combination in a medical safety injector according to a third embodiment of this invention;
Fig. 9 is a cross-sectional view of a plunger combination in a medical safety injector according to a fourth embodiment of this invention;
Fig. 10 is a cross-sectional view of a plunger combination in a medical safety injector according to a fifth embodiment of this invention;
Fig. 11 is a schematic view illustrating how a retractable plunger retracts into a hollow shank in the medical safety injector according to the fifth embodiment of this invention;
Fig. 12 is a perspective view of a retractable plunger having a roughened portion in a medical safety injector according to a sixth embodiment of this invention;
Fig. 13 is an exploded view of a medical safety injector according to a seventh embodiment of this invention; and
Fig. 14 is an exploded view of a medical safety injector according to an eighth embodiment of this invention.

### DETAILED DESCRIPTION OF THE INVENTION

Hereinafter, a detailed description of this invention will be provided below with reference to embodiments thereof. Other advantages and features of this invention will be readily appreciated by those skilled in the art upon reviewing this disclosure. However, this invention may also be implemented or applied as other embodiments, and various details in this specification may be modified and altered from different viewpoints and based on different applications without departing from the scope of this invention as defined in claim 1.

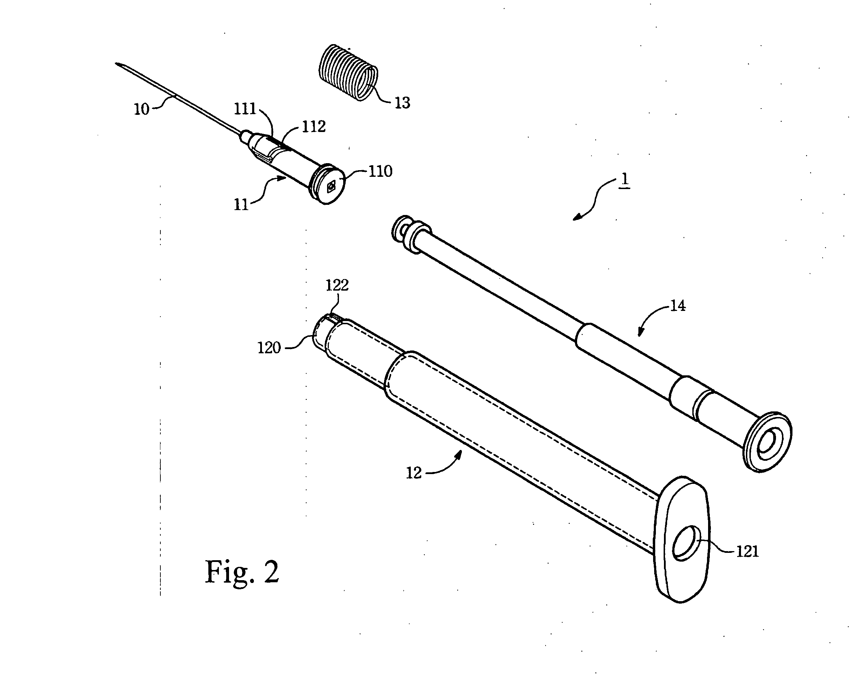

Figs. 2, 3 and 4 provide perspective and cross-sectional views of various parts of an automatically retractable medical safety injector and a plunger combination thereof according the present invention. The automatically retractable medical safety injector is used for intramuscular injection, hypodermic injection, intravenous injection or drawing blood and allows for only a single use. It should be noted that, all the above drawings are simplified schematic views intended to illustrate a preferred embodiment of this invention only schematically. Components associated with this invention as shown in the above drawings are not intended to represent exact forms in practical implementations; rather, the number, shapes and dimensions of the components can be designed as desired in practical implementations, wherein arrangement of the components is often much more complex than depicted in the drawings.

Referring to Fig. 2, an automatically retractable medical safety injector 1 according to a first embodiment of this invention comprises: a needle hub 11 connected with a needle 10; a hollow barrel 12; and a plunger combination 14 disposed within the hollow barrel 12. The hollow barrel 12 is capable of retaining the needle hub 11 and, after completion of injection, guiding the needle hub 11 to retract in a direction opposite to an injection direction by virtue of at least one helical retractile spring 13.

In more detail, one end of the needle hub 11 receives the needle 10 that extends through a central portion of the needle hub 11, while the other end of the needle hub 11 is provided with a disc stopper 110 for the compressed helical retractile spring 13 to abut against. At proper locations between the two ends of the needle hub 11, a plurality of positioning grooves 111 and a plurality of U-shaped retraction slideways 112 connected with the positioning grooves 111 are provided. The positioning grooves 111 are adapted to be snap-fitted with a front end 120 of the hollow barrel 12. When the helical retractile spring 13 is decompressed, the disengaged needle hub 11 will retract in the direction opposite to the injection direction along the U-shaped retraction slideways 112. A distance by which the needle hub 11 retracts can be controlled by the U-shaped retraction slideways 112 to prevent the needle 10 from being exposed.

Further, the hollow barrel 12 has the front end 120 and a back end 121. As shown in Fig. 2, the front end 120 of the hollow barrel 12 is provided with a plurality of slightly resilient catches 122 facing a center of the circle. In a normal unused status and during injection, the catches 122 are stably snap-fitted in the positioning grooves 111 of the needle hub 11. When a user applies a force towards the needle after completion of injection, the catches 122 at the front end 120 of the hollow barrel 12 are indirectly forced to expand in a resilient way and thus disengage from the needle hub 11, allowing the needle hub 11 to retract into the hollow barrels 12.

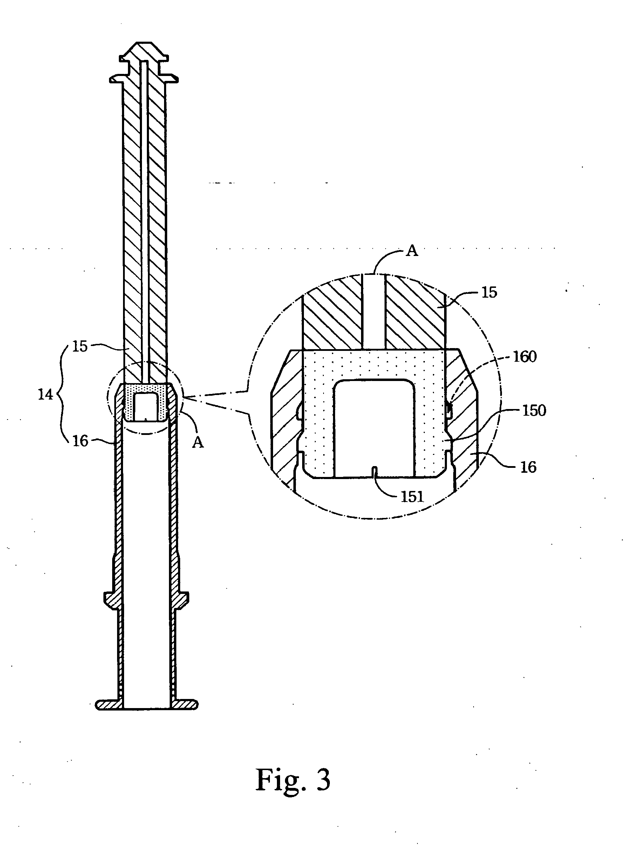

Referring to Fig. 3, the automatically retractable medical safety injector 1 according to the first embodiment of this invention is characterized mainly by the design of the plunger combination 14. As shown in the drawing, the plunger combination 14 has a two-piece structure consisting of a retractable plunger 15 and a hollow shank 16 which are injection molded separately and then put together. A bottom portion of the retractable plunger 15 is muff-coupled to the hollow shank 16 and, as shown in a partial enlarged view (i.e., a muff-coupled region A) in Fig. 3, an exterior sidewall of the retractable plunger 15 is formed with a plurality of protrusions 150, while an interior sidewall of the hollow shank 16 is formed with a plurality of recesses 160 corresponding to the protrusions 150. In a normal unused status or during an injection process of the plunger combination 14, the protrusions 150 of the retractable plunger 15 are snap-fitted tightly in the recesses 160 of the hollow shank 16. Only when the user applies a force to disengage the protrusions 150 from the recesses 160 after completion of injection, will the retractable plunger 15 retract into the hollow shank 16 and free space originally occupied by the retractable plunger 15 in the hollow barrel 12, so as to receive the retracted needle hub 11 (not shown in Fig. 3; please refer to Fig. 2 for the needle hub 11).

Additionally, the retractable plunger 15 or the hollow shank 16 shown in Fig. 3 is injection molded from a slightly resilient material such as polyethylene (PE), polyvinyl chloride (PVC) or rubber, or a transparent rigid plastic such as polypropylene (PP) or those from the AN series. By virtue of stiffness provided by the transparent rigid plastic and the resilient material, a desirable injecting force can be maintained by the retractable plunger 15 and the hollow shank 16. In this embodiment, a main body of the retractable plunger 15 may be formed as a cross rib or a tubular column.

To attain equilibrium of stress on the plunger, a variation of the first embodiment of this invention is proposed. In this variation, at a core portion of the muff-coupled region A where the retractable plunger 15 is muff-coupled with the hollow shank 16, the retractable plunger 15 is provided with one or more lateral stress-adjustment cutouts 151, each of which may be a cavity, a blind hole or a through-hole. Generally, the lateral stress-adjustment cutout 151 is longitudinally proximate to but still keeps away from where the protrusions 150 are formed on the exterior sidewall of the retractable plunger 15. Therefore, when a force is further applied to the plunger combination 14 after completion of injection, the lateral stress-adjustment cutout 151 is allowed to deform so as to disengage the protrusions 150 from the recesses 160.

Furthermore, the protrusions 150 in this embodiment may be protruding spots formed together with the retractable plunger 15 during injection molding. While six protruding spots arranged in symmetry are illustrated as a preferred embodiment herein, no substantial limitation is imposed on the shape, size, quantity and arrangement of the protruding spots. Annular protruding ribs, for example, are also contemplated as within the equivalent scope of the protrusions 150 described in this embodiment.

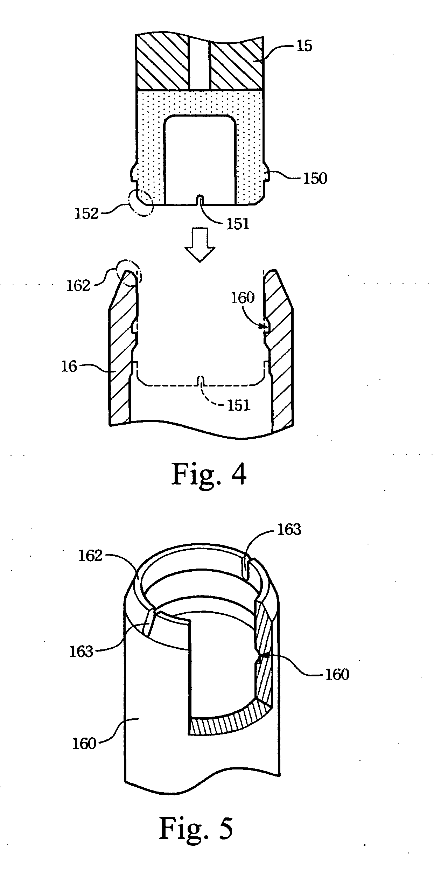

A structure of the hollow shank 16 is shown in Fig. 4. To accomplish muff-coupling and tight snap-fitting with the retractable plunger 15, the hollow shank 16 has a shank body whose interior sidewall is formed with the plurality of recesses 160, which are located near the muff-coupled region where the hollow shank 16 is muff-coupled with the retractable shank 15, and can be snap-fitted with the protrusions 150 correspondingly. These recesses 160 may be grooves, cavities, or partial or complete annular concave ribs, and may have a C-shaped, angled circular or irregular cross section. In other words, any configurations that can be snap-fitted tightly with corresponding protrusions 150 of the retractable plunger 15 are contemplated as within the equivalent scope of the recesses 160 of this embodiment, although complete annular concave ribs are illustrated herein as a preferred embodiment of the recesses 160, so that the retractable plunger 15 is allowed to rotate during assembly without being restricted by geometric positions of the protrusions 150.

The muff-coupled region A between the retractable plunger 15 and the hollow shank 16 of the plunger combination 14 is shown in detail in the enlarged view of Fig. 4. A bottom edge 152 of a plunger body of the retractable plunger 15 below the protrusions 150 is designed with a rounded shape, while an inner edge 162 of an opening of the hollow shank 16 is formed with an arcuate, rounded or bevel surface, so that an inner diameter of the edge 162 of the opening of the hollow shank 16 is equal to or slightly larger than a diameter of the bottom edge of the plunger body of the retractable plunger 15. As a result, when the retractable plunger 15 and the hollow shank 16 are disengaged from each other after completion of injection, the retractable plunger 15 can slide smoothly into the hollow shank 16 to complete a retracting process successfully.

In practical mass production of the plunger combination 14, the retracting force and tensile strength of the retractable plunger 15 and the hollow shank 16 shall conform to relevant mechanics testing standards. Hence, as shown in Fig. 5, a further variation of the first embodiment of this invention is disclosed, wherein at least one stress-adjustment cutout 163 is further provided at a beveled edge 162 of the hollow shank 16. The stress-adjustment cutout 163 keeps away longitudinally from the recesses 160 on the interior sidewall of the hollow shank 16. Besides, the stress-adjustment cutout 163 may also be, for example, a cavity or a blind hole.

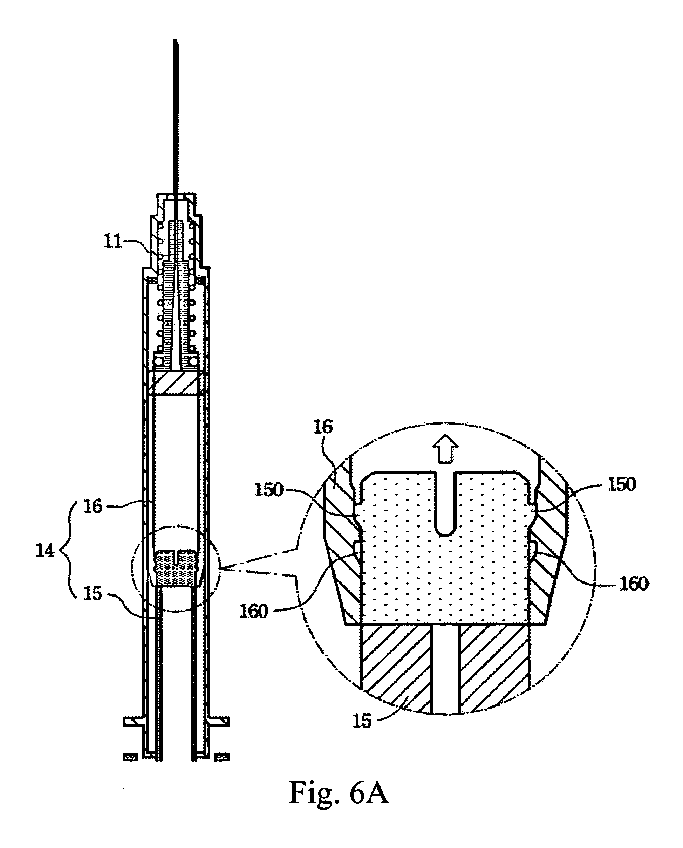

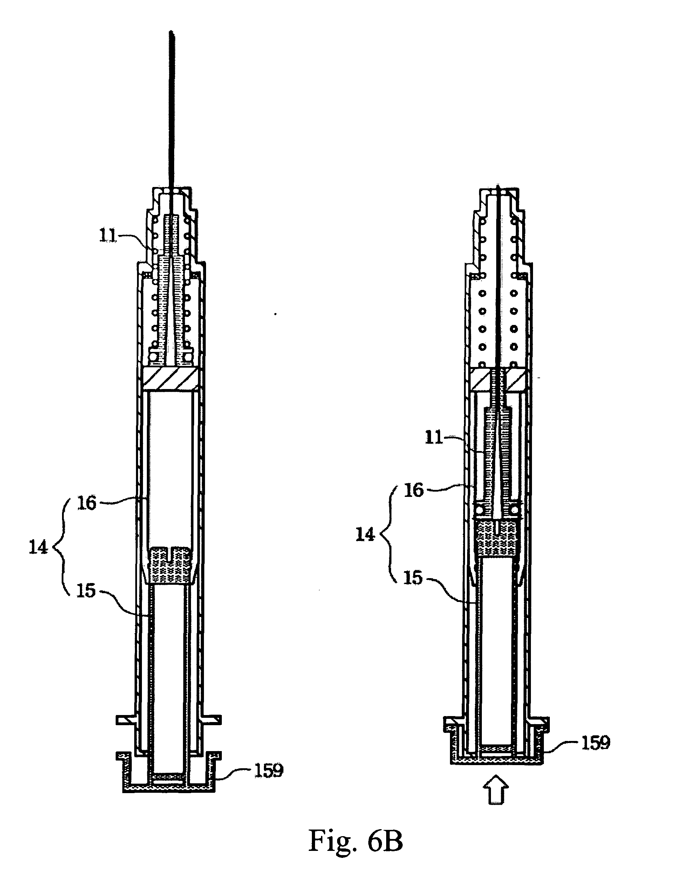

In the plunger combination 14 of the medical safety injector 1 of this invention, a relative position of the hollow shank 16 and the retractable plunger 15 in a fore-and-aft direction can be reversed as desired without substantial limitation. In addition to the configuration shown in Fig. 3 where the retractable plunger 15 is disposed proximate to the needle hub 11 so that a retracting direction of the retractable plunger 15 after injection is consistent with that of the needle hub 11 after being detached, the retractable plunger 15 may also be disposed at a rear section of the hollow barrel 12 away from the needle hub 11, as in another variation of this embodiment. Referring to Fig. 6A, a plunger combination 14 of this embodiment also has a two-piece structure consisting of a retractable plunger 15 and a hollow shank 16. An exterior sidewall of the retractable plunger 15 is formed with a plurality of protrusions 150, while an interior sidewall of the hollow shank 16 is formed with a plurality of recesses 160 corresponding to the protrusions 150. In a normal unused status or during an injection process of the plunger combination 14, the protrusions 150 of the retractable plunger 15 and the recesses 160 of the hollow shank 16 are snap-fitted together tightly. However, the embodiment shown in Fig. 6A is different in that a relative position of the hollow shank 16 and the recesses 160 in the fore-and-aft direction is reversed. Only when a user applies a force to disengage the protrusions 150 on the interior sidewall of an upper portion of the retractable plunger 15 from the recesses 160 after completion of injection, will the retractable plunger 15 retract into the hollow shank 16. As shown in Fig. 6B, the retractable plunger 15 retracts into the hollow shank 16 in a direction opposite to a retracting direction of the detached needle hub 11. Furthermore, to attain better operability during single-handed injection of the plunger combination 14 having the retractable plunger 15 disposed at the rear section of the hollow barrel 12, additional extended wings 159 may be provided at both sides of a distal end of the hollow barrel 12 and at both sides of a bottom portion of the retractable plunger 15 in a preferred implementation of this embodiment, so as to control a retraction distance of the retractable plunger 15, thereby reserving sufficient space in the hollow barrel 12 for receiving the needle hub 11.

All implementations described herein are preferred implementations of the first embodiment of this invention but not intended to limit the scope of this invention. Therefore, any two-piece plunger assemblies formed by a hollow shank and a retractable plunger muff-coupled together, regardless of the sequence in which they are muff-coupled, the way they are snap-fitted or the way they are connected in segments, are contemplated as within the equivalent scope of this invention that is readily conceivable and can be reasonably enlarged. Meanwhile, in order to cope with various possible strengths or angles with or at which a force is applied by a single hand during an injection process, a further reinforcing design is provided for the retractable plunger and the hollow shank of the plunger combination in the medical safety injector of this invention.

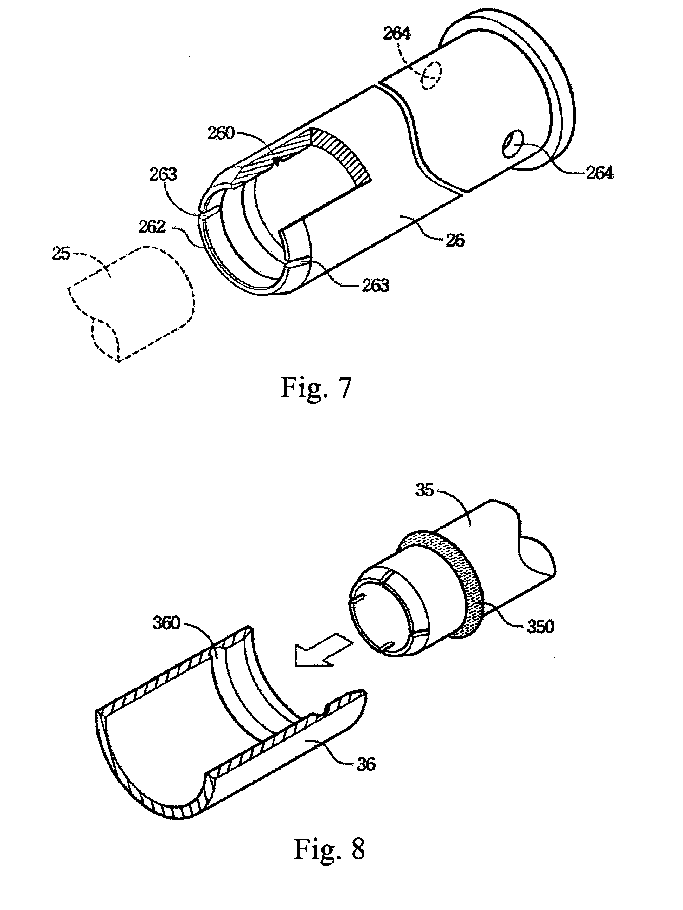

Figs. 7 to 14 illustrate variations of embodiments of the medical safety injector and the plunger combination thereof of this invention. Since the following embodiments are all substantially the same as the first embodiment described above except for slight differences in their respective characteristic portions, description is provided only for features of the second to eighth embodiments that are different from the first embodiment, whereas components identical to those of the first embodiment will be omitted from the description.

Fig. 7 shows a medical safety injector and a plunger combination thereof according to a second embodiment of this invention. As shown therein, in the plunger combination of this embodiment, one or more vent holes 264 are additionally provided in a sidewall of a hollow shank 26 distal from a muff-coupled region (as indicated by A in Fig. 3). These vent holes 264 facilitate a user's operation by preventing the user from feeling air resistance on his/her thumb when a retractable plunger 25 is retracting into the hollow shank 26. In this embodiment, annular concave ribs 260 replace the recesses 160 of the first embodiment while an edge 262 and stress-adjustment cutouts 263 are the same as the edge 162 and the stress-adjustment cutouts 163 of the first embodiment respectively.

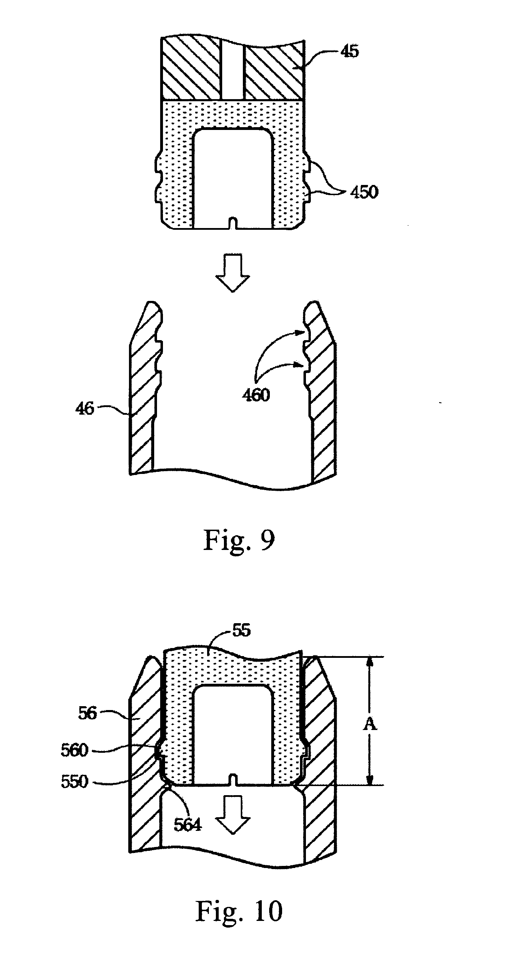

Referring to Fig. 8, in a medical safety injector and a plunger combination thereof according to a third embodiment of this invention, a lateral annular protruding rib 350 with a continuous or discontinuous profile is provided on an exterior sidewall of a retractable plunger 35 to replace the protrusions 150 in the first embodiment. Meanwhile, an annular concave rib 360 with a continuous or discontinuous profile or one or more annular grooves 360 with continuous or discontinuous profiles are correspondingly formed on a hollow shank 36 to replace the recesses 160 of the first embodiment. In addition to the one-row design, a multiple-row design may also be adopted for the annular protruding rib 350 and the annular concave rib 360, as in a medical safety injector and a plunger combination thereof according to a fourth embodiment of this invention illustrated in Fig. 9.

As shown in Fig. 9, in the fourth embodiment of this invention, multiple rows of annular protruding ribs 450 adjacent to or spaced apart from each other with continuous or discontinuous lateral profiles are formed on an exterior sidewall of a retractable plunger 45 of the plunger combination to replace the protrusions 150 of the first embodiment. Furthermore, at corresponding locations on an interior sidewall of a hollow shank 46, multiple rows of annular concave ribs 460 with continuous or discontinuous profiles or multiple rows of annular grooves 460 with continuous or discontinuous profiles are formed adjacent to or spaced apart from each other to replace the recesses 160 of the first embodiment.

On the other hand, in order for the retractable plunger to conform to mechanics requirements of tension strength and retracting force during an injection process, further improvements on a portion of the hollow shank below the recesses or annular concave ribs or annular grooves are also disclosed in this invention. Fig. 10 shows a medical safety injector and a plunger combination thereof according to a fifth embodiment of this invention. As shown therein, in the muff-coupled region A of the plunger combination, a plurality of stoppers 564 are formed on an interior sidewall of a hollow shank 56 near a bottom edge of a recess or annular concave rib or annular groove 560 along a direction in which a retractable plunger 55 retracts. By using the stoppers 564 to enhance a counter force against the retractable plunger 55 when injection is performed, the retractable plunger 55 and the hollow shank 56 can be joined together more securely. The stoppers 564 may be protrusions integrally formed on the hollow shank 56 while the hollow shank 56 is injection molded, or blocks additionally provided. An extended surface of each stopper 564 may be kept at a right angle to a longitudinal axis of the hollow shank 56, or inclined at an angle consistent with a retracting direction of the retractable plunger 55.

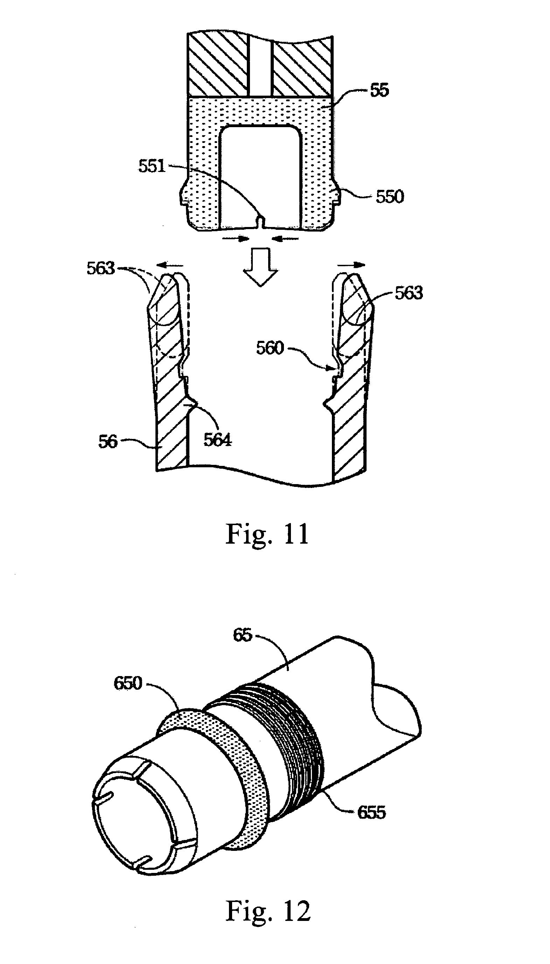

In a preferred implementation of the fifth embodiment of this invention, the stoppers 564 are designed with a downward inclined angle consistent with the retracting direction of the retractable plunger 55. Hence, as shown in Fig. 11, upon completion of injection, a user may apply a force to the plunger combination so that the protrusions 550 of the retractable plunger 55 contract laterally inwards towards a stress-adjustment cutout 551 while retracting into the hollow shank 56. At this point, the downward inclined angle of the stoppers 564 will guide the retractable plunger 55 smoothly into the hollow shank 56. Further, as the hollow shank 56 is slightly resilient, application of force to the plunger combination also causes stress-adjustment cutouts 563 at an edge of the hollow shank 56 to expand laterally outwards instantaneously, so that the retractable plunger 55 can retract into the hollow shank 56 more easily.

Fig. 12 illustrates a medical safety injector and a plunger combination thereof according to a sixth embodiment of this invention. As shown therein, in this embodiment, a roughened portion 655 comprising, for example, an accidented region, transverse striations or a unsmoothed grainy surface, is formed on an exterior sidewall of a retractable plunger 65 above a protrusion 650, in order to counteract or buffer an excessively strong force applied by a user when pushing the plunger. This will prevent the retractable plunger 65 from retracting into a hollow shank (not shown) before injection is completed and thereby avoid injection failure.

The automatically retractable medical safety injector and the plunger combination thereof according to this invention have a two-piece split configuration, in which the retractable plunger and the hollow shank are injection molded separately from a flexible material (e.g., plastic or rubber) and then assembled together. This may lower the risk of inadvertent damage to the plunger due to impact during assembly, and reduce injection faults attributed to damage of the plunger prior to unpacking and during use. Moreover, as the two pieces of the plunger are molded separately and then put together, the plunger may have a relaxed requirements of mold precision because it is no more necessary to consider force equilibrium at the connecting joint as in the prior art plunger integrally formed with the breakable connection. Hence, the development cost of molds is reduced while reliability and quality stability are improved.

On the other hand, the two-piece plunger combination exhibits better force equilibrium features than plungers with breakable connections of the existing syringes in terms of lateral shear strength. Therefore, when a patient performs an injection with a single hand or when a medical care worker performs an injection at a site difficult to access, the injection operation will not be affected by restrictions in the injection angle or posture.

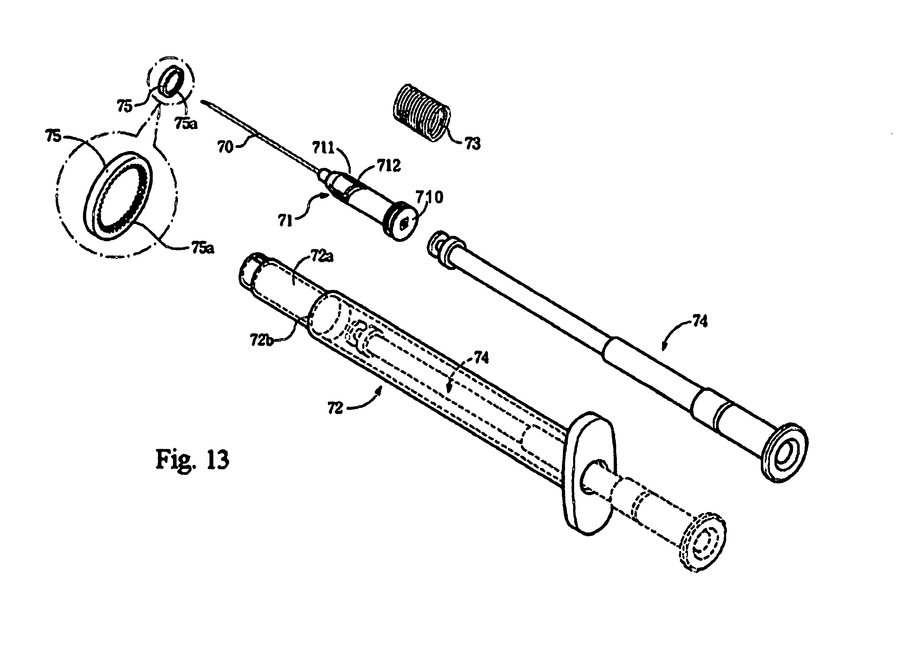

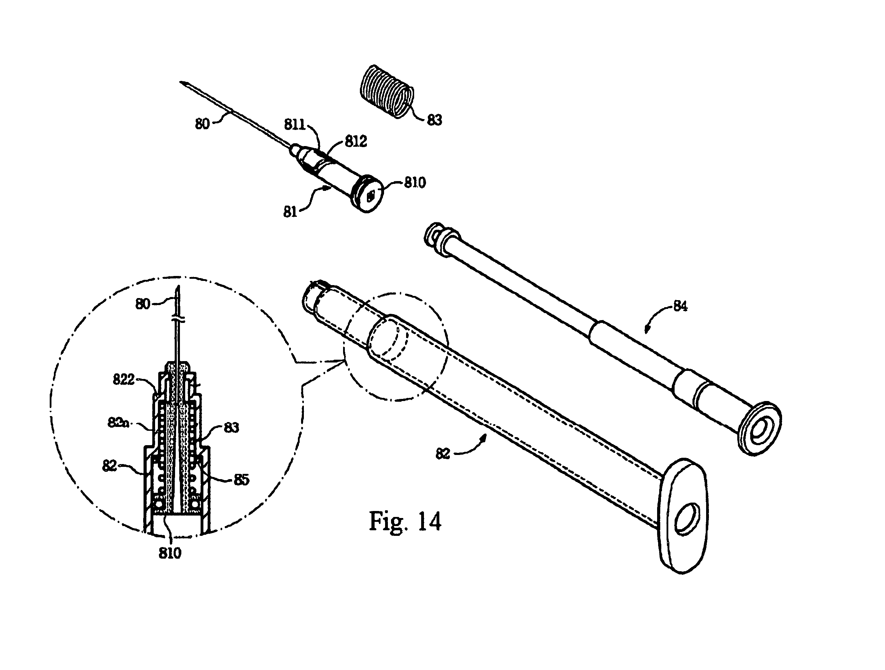

Particularly, in order to improve production pass rate and operability, the medical safety injector and the plunger combination thereof according to this invention also takes on a best design of the needle hub, as described hereunder in more detail with reference to a seventh and an eighth embodiment shown in Figs. 13 and 14 respectively.

Fig. 13 shows a medical safety injector and a plunger combination 74 thereof according to the seventh embodiment of this invention. As shown therein, in order to improve the pass rate of retracting operation of the needle hub, a narrow barrel portion 72a having a diameter smaller than a diameter of a hollow barrel 72 is formed integrally in a front section of the hollow barrel 72 to receive a compressed helical spring 73 and a front half of a needle hub 71. In an opening 72b of the hollow barrel 72 where the hollow barrel 72 is connected with the narrow barrel portion 72a, at least one movable or fixed ring stopper 75 is accommodated to prevent the needle hub 71 from slipping out under an injection force and to guide the needle hub 71 to retract upon completion of injection. The movable or fixed ring stopper 75 is centrally provided with a through-hole 75a for a needle 70 and a portion of the needle hub 71 to pass through, wherein an area of the through-hole 75a of the ring stopper 75 is smaller than an area of a disc stopper 710 of the needle hub 71, so as to stop the disc stopper 710 of the needle hub 71 and maintain the needle hub 71 at a best preset retracting position. The movable or fixed ring stopper 75 may be one or more rings having a smooth interior wall or an interior wall with regular or irregular teeth. A ring having a through-hole formed with radial teeth is illustrated herein as a preferred implementation of this embodiment. The needle 70, needle hub 71, positioning grooves 711, U-shaped retraction slideways 712 and helical retractile spring 73 in this embodiment are just the same as the needle 10, needle hub 11, positioning grooves 111, U-shaped retraction slideways 112 and helical retractile spring 13 of the first embodiment respectively.

Apart from the additional movable or fixed ring stopper 75 provided at the opening 72b joining the hollow barrel 72 and the narrow barrel portion 72a, according to the eighth embodiment of this invention shown in Fig. 14, a bottleneck portion 85 is formed integrally at an opening where a narrow barrel portion 82a of a hollow barrel 82 is connected with the hollow barrel 82 during injection molding of the hollow barrel 82. The bottleneck portion 85 is centrally formed with a through-hole having an area smaller than that of a disc stopper 810, so that the bottleneck portion 85 allows only a needle 80 and a front half of a needle hub 81 to pass through but stops the disc stopper 810, thereby accomplishing the same function of preventing the needle hub from slipping out as described in the previous embodiment. A plunger combination 84, helical retractile spring 83, positioning grooves 811, U-shaped retraction slideways 812 and catches 822 of this embodiment are just the same as the plunger combination 14, helical retractile spring 13, positioning grooves 111, U-shaped retraction slideways 112 and catches 122 of the first embodiment respectively.

The above embodiments are provided only to demonstrate principles and functions of the present invention and not intended to limit the scope of the present invention. Various changes in form and details may be made by those of ordinary skill in the art without departing from the scope of the present invention as defined by the following claims.

## Claims

1. A medical safety injector (1), comprising a needle hub (11, 11', 71, 81) connected with a needle (10, 10', 70, 80), a hollow barrel (12, 12', 72, 82) for retaining the needle hub (11, 11', 71, 81) and, after the medical safety injector (1) has completed an injection, guiding the needle hub (11, 11', 71, 81) to retract into the hollow barrel (12, 12', 72, 82); and the medical safety injector (1) is **characterized in that** the medical safety injector (1) includes a plunger combination (14, 74, 84) disposed within the hollow barrel (12, 12', 72, 82), wherein the plunger combination (14, 74, 84) has a retractable plunger (15, 25, 35, 45, 55, 65) and a hollow shank (16, 26, 36, 46, 56);
the needle hub (11) has two ends, and one end of the needle hub (11) receives the needle (10) that extends through a central portion of the needle hub (11), while the other end of the needle hub (11) is provided with a disc stopper (110), and a plurality of positioning grooves (111) and a plurality of U-shaped retraction slideways (112) connected with the positioning grooves (11) are provided at proper locations between two ends of the needle hub (11);
the hollow barrel (12) has a front end (120) and a back end (121), and a plurality of slightly resilient catches (122) are provided at the front end (120), and the catches (122) are stably snap-fitted in the positioning grooves (111) of the needle hub (11) in a normal unused status and during the injection;
at least one helical retractile spring (13) is provided to abut against the disc stopper (110) of the needle hub (11) and is compressed inside a space of the hollow barrel (12, 12', 72, 82) in a normal unused status and during the injection;
wherein when a force is applied to the plunger combination (14, 74, 84) and therefore toward the needle (10) after completion of the injection, the at least one protrusion (150, 550, 650) is disengaged from the at least one recess (160) due to the force, and the catches (122) at the front end (120) of the hollow barrel (12) are indirectly forced to expand in a resilient way and thus disengage from the needle hub (11), and the retractable plunger (15, 25, 35, 45, 55, 65) is forced to slide into the hollow shank (16, 26, 36, 46, 56), and the space of the hollow barrel (12, 12', 72, 82) accommodating the compressed helical retractile spring (13) expands to allow the compressed helical retractile spring (13) to decompress and allow the needle hub (11, 11', 71, 81) to be retracted into the hollow barrel (12, 12', 72, 82) in a direction opposite to an direction of the injection along the U-shaped retraction slideways (112);
**characterised in that** the retractable plunger (15, 25, 35, 45, 55, 65) and the hollow shank (16, 26, 36, 46, 56) are separately injection-molded from slightly resilient material and put together to allow a bottom portion of the retractable plunger (15, 25, 35, 45, 55, 65) be muff-coupled to a top portion of the hollow shank (16, 26, 36, 46, 56) and thus form a muff-coupled region (A), and the retractable plunger (15, 25, 35, 45, 55, 65) has an exterior sidewall formed with at least one protrusion (150, 550, 650) in the muff-coupled region (A) for being correspondingly tightly snap-fitted with at least one recess (160) formed on an interior sidewall of the hollow shank (16, 26, 36, 46, 56) in the muff-coupled region (A) in a normal unused status or during the injection, and wherein the retractable plunger (15, 25, 35, 45, 55, 65) includes at least one stress-adjustment cutout (151, 551) provided at a core portion of the muff-coupled region (A) or the hollow shank (16, 26, 36, 46, 56) has an edge (162, 262) provided with at least one stress-adjustment cutout (163,263,563) in the muff-coupled region (A).

2. The medical safety injector (1) of claim 1, wherein the at least one protrusion (150, 550, 650) is selected from the group consisting of a protruding spot formed integrally on the retractable plunger (15, 25, 35, 45, 55, 65), one or more rows of lateral annular protruding ribs (350, 450) adjacent to each other, and one or more rows of lateral annular protruding ribs (350, 450) spaced apart from each other.

3. The medical safety injector (1) of claim 2, wherein any one of the lateral annular protruding ribs (350, 450) has a continuous or discontinuous profile.

4. The medical safety injector (1) of claim 1, wherein the retractable plunger (15, 25, 35, 45, 55, 65) has a plunger body whose bottom edge (152) is rounded.

5. The medical safety injector (1) of claim 1, wherein the at least one recess (160) is one of a groove and a cavity.

6. The medical safety injector (1) of claim 1, wherein the at least one recess (160) is selected from the group consisting of one or more rows of annular concave ribs (260, 360, 460, 560) adjacent to each other, one or more rows of annular concave ribs (260, 360, 460, 560) spaced apart from each other, one or more rows of annular grooves (360, 460, 560) adjacent to each other, and one or more rows of annular grooves (360, 460, 560) spaced apart from each other.

7. The medical safety injector (1) of claim 6, wherein any one of the annular concave ribs (260, 360, 460, 560) or any one of the annular grooves (360, 460, 560) has a continuous profile.

8. The medical safety injector (1) of claim 6, wherein any one of the annular concave ribs (260, 360, 460, 560) or any one of the annular grooves (360, 460, 560) has a C-shaped or an angled circular cross section.

9. The medical safety injector (1) of claim 1, wherein the hollow shank (16, 26, 36, 46, 56) has a sidewall provided with at least one vent hole (264) distal from the muff--coupled region (A).

10. The medical safety injector (1) of claim 1, wherein the at least one stress-adjustment cutout (151,551) is one of a cavity, a blind hole and a through-hole.

11. The medical safety injector (1) of claim 1, wherein the hollow barrel (12, 12', 72, 82) has a front section which is near the needle hub (11, 11', 71, 81) and formed integrally with a narrow barrel portion (72a, 82a) having a diameter smaller than a diameter of the hollow barrel (12, 12', 72, 82), and the hollow barrel (12, 12', 72, 82) has an opening (72b) which is connected with the narrow barrel portion (72a, 82a) and provided with at least one ring stopper (75) to prevent the needle hub (11, 11', 71, 81) from slipping out and to guide the needle hub (11, 11', 71, 81) to retract after completion of the injection.

12. The medical safety injector (1) of claim 1, wherein the hollow barrel (12, 12', 72, 82) has a front section which is near the needle hub (11, 11', 71, 81) and formed integrally with a narrow barrel portion (72a, 82a) having a diameter smaller than a diameter of the hollow barrel (12, 12', 72, 82), and the hollow barrel (12, 12', 72, 82) has an opening (72b) which is connected with the narrow barrel portion (72a, 82a) and formed integrally with at least one bottleneck portion (85) to prevent the needle hub (11, 11', 71, 81) from slipping out and to guide the needle hub (11, 11', 71, 81) to retract after completion of the injection.

## Patentansprüche

1. Medizinisches Sicherheitsinjektionsgerät (1) mit einem Nadelsitz (11, 11', 71, 81), der mit einer Nadel (10, 10', 70, 80) verbunden ist, einem Hohlzylinder (12, 12', 72, 82) zur Aufnahme des Nadelsitzes (11, 11', 71, 81) und Führung des Nadelsitzes (11, 11', 71, 81) beim Zurückziehen in den Hohlzylinder (12, 12', 72, 82) nach Beendigung einer Injektion mit dem medizinischen Sicherheitsinjektionsgerät (1), wobei das medizinische Sicherheitsinjektionsgerät (1) daurch gekennzeichnet ist, dass das medizinische Sicherheitsinjektionsgerät (1) eine Kolbenkombination (14, 74, 84) umfasst, die in dem Hohlzylinder (12, 12', 72, 82) angeordnet ist, wobei die Kolbenkombination (14, 74, 84) einen einziehbaren Kolben (15, 25, 35, 45, 55, 65) und einen Hohlschaft (16, 26, 36, 46, 56) beinhaltet,
wobei der Nadelsitz (11) zwei Enden umfasst, wobei ein Ende des Nadelsitzes (11) die Nadel (10) aufnimmt, welche sich durch einen mittleren Abschnitt des Nadelsitzes (11) erstreckt, während das andere Ende des Nadelsitzes (11) einen Scheibenanschlag (110) aufweist, wobei eine Vielzahl an Positionierrillen (111) und eine Vielzahl an U-förmigen Einzugsführungsbahnen (112), welche mit den Positionierrillen (11) verbunden sind, an geeigneten Positionen zwischen den beiden Enden des Nadelsitzes (11) vorgesehen sind,
wobei der Hohlzylinder (12) ein vorderes Ende (120) und ein hinteres Ende (121) aufweist, wobei eine Vielzahl an leicht federnden Rasten (122) an dem vorderen Ende (120) bereitgestellt ist, wobei die Rasten (122) stabil in die Positionierrillen (111) des Nadelsitzes (11) in einem normalen ungebrauchten Zustand und während einer Injektion einrasten,
wobei wenigstens eine einziehbare Schraubenfeder (13) vorhanden ist, die an den Scheibenanschlag (110) des Nadelsitzes (11) anschlägt und in einen Leerraum des Hohlzylinders (12, 12', 72, 82) in einem normalen ungebrauchten Zustand und während einer Injektion gedrückt wird,
wobei, wenn nach der Beendigung der Injektion eine Kraft auf die Kolbenkombination (14, 74, 84) und demzufolge in Richtung der Nadel (10) wirkt, der wenigstens eine Vorsprung (150, 550, 650) aus der wenigstens einen Aussparung (160) durch die Krafteinwirkung gelöst wird und die Rasten (122) an dem vorderen Ende (120) des Hohlzylinders (12) indirekt dazu gebracht werden, sich in elastischer Weise auszudehnen und sich dadurch von dem Nadelsitz (11) lösen, wodurch der einziehbare Kolben (15, 25, 35, 45, 55, 65) dazu gebracht wird, in den Hohlschaft (16, 26, 36, 46, 56) hineinzugleiten, und sich der Leerraum des Hohlzylinders (12, 12', 72, 82), in welchem die zusammengedrückte einziehbare Schraubenfeder (13) aufgenommen ist, vergrößert, sodass sich die zusammengedrückte einziehbare Schraubenfeder (13) ausdehnen kann, wodurch der Nadelsitz (11, 11', 71, 81) in einer Richtung entgegen der Injektionsrichtung entlang der U-förmigen Einzugsführungsbahnen (112) in den Hohlzylinder (12, 12', 72, 82) eingezogen wird,
**dadurch gekennzeichnet, dass** der einziehbare Kolben (15, 25, 35, 45, 55, 65) und der Hohlschaft (16, 26, 36, 46, 56) getrennt voneinander aus einem leicht elastischem Material spritzgegossen und zusammengesetzt sind, wobei ein unterer Abschnitt des einziehbaren Kolbens (15, 25, 35, 45, 55, 65) mit einem oberen Abschnitt des Hohlschafts (16, 26, 36, 46, 56) mittels einer Muffenkupplung verbunden ist, wobei ein Muffenkupplungsbereich (A) ausgebildet wird, und wobei der einziehbare Kolben (15, 25, 35, 45, 55, 65) eine äußerte Seitenwand umfasst, die mit wenigstens einem Vorsprung (150, 550, 650) in dem Muffenkupplungsbereich (A) ausgebildet ist, der in einem normalen ungebrauchten Zustand oder während einer Injektion mit wenigstens einer Aussparung (160), die an einer inneren Seitenwand des Hohlschafts (16, 26, 36, 46, 56) in dem Muffenkupplungsbereich (A) ausgebildet ist, entsprechend eine feste Rastverbindung ausbildet, und wobei der einziehbare Kolben (15, 25, 35, 45, 55, 65) wenigstens einen Druckanpassungsausschnitt (151, 551) umfasst, der an einem Kernabschnitt des Muffenkupplungsbereichs (A) vorgesehen ist, oder der Hohlschaft (16, 26, 36, 46, 56) einen Rand (162, 262) aufweist, der wenigstens einen Druckanpassungsausschnitt (163,263,563) in dem Muffenkupplungsbereich (A) umfasst.

2. Medizinisches Sicherheitsinjektionsgerät (1) nach Anspruch 1, wobei der wenigstens eine Vorsprung (150, 550, 650) aus einer Gruppe ausgewählt ist, die einen vorstehenden Stift, der einstückig an dem einziehbaren Kolben (15, 25, 35, 45, 55, 65) ausgebildet ist, eine oder mehrere Reihen von einander benachbarten, seitlich ringförmig hervorstehenden Rippen (350, 450), und eine oder mehrere Reihen von seitlich ringförmig hervorstehenden Rippen (350, 450), welche voneinander getrennt sind, umfasst.

3. Medizinisches Sicherheitsinjektionsgerät (1) nach Anspruch 2, wobei eine der seitlich ringförmig hervorstehenden Rippen (350, 450) ein kontinuierliches oder unterbrochenes Profil aufweist.

4. Medizinisches Sicherheitsinjektionsgerät (1) nach Anspruch 1, wobei der einziehbare Kolben (15, 25, 35, 45, 55, 65) einen Kolbenkörper aufweist, dessen unterer Rand (152) abgerundet ist.

5. Medizinisches Sicherheitsinjektionsgerät (1) nach Anspruch 1, wobei die wenigstens eine Aussparung (160) eine Nut oder ein Hohlraum ist.

6. Medizinisches Sicherheitsinjektionsgerät (1) nach Anspruch 1, wobei die wenigstens eine Aussparung (160) aus einer Gruppe ausgewählt ist, die eine oder mehrere Reihen von zueinander benachbarten, ringförmigen konkaven Rippen (26D, 360, 460, 560), eine oder mehrere Reihen von ringförmigen konkaven Rippen (260, 360, 460, 560), die voneinander getrennt sind, eine oder mehrere Reihen von zueinander benachbarten, ringförmigen Nuten (360, 460, 560) und eine oder mehrere Reihen von ringförmigen Nuten (360, 460, 560), die voneinander getrennt sind, umfasst.

7. Medizinisches Sicherheitsinjektionsgerät (1) nach Anspruch 6, wobei eine der ringförmigen konkaven Rippen (260, 360, 460, 560) oder eine der ringförmigen Nuten (360, 460, 560) ein kontinuierliches Profil aufweist.

8. Medizinisches Sicherheitsinjektionsgerät (1) nach Anspruch 6, wobei eine der ringförmigen konkaven Rippen (260, 360, 460, 560) oder eine der ringförmigen Nuten (360, 460, 560) einen C-förmigen oder einen abgewinkelt ringförmigen Querschnitt aufweist.

9. Medizinisches Sicherheitsinjektionsgerät (1) nach Anspruch 1, wobei der Hohlschaft (16, 26, 36, 46, 56) eine Seitenwand umfasst, die wenigstens eine Lüftungsöffnung (264) beinhaltet, die sich entfernt von dem Muffenkupplungsbereich (A) befindet.

10. Medizinisches Sicherheitsinjektionsgerät (1) nach Anspruch 1, wobei der wenigstens eine Druckanpassungsausschnitt (151,551) entweder ein Hohlraum, eine Sackbohrung oder eine Durchgangsbohrung ist.

11. Medizinisches Sicherheitsinjektionsgerät (1) nach Anspruch 1, wobei der Hohlzylinder (12, 12', 72, 82) einen vorderen Teil umfasst, der in der Nähe des Nadelsitzes (11, 11', 71, 81) und einstückig mit einem schmalen Schaftabschnitt (72a, 82a) ausgebildet ist, dessen Durchmesser kleiner ist als der Durchmesser des Hohlzylinders (12, 12', 72, 82), wobei der Hohlzylinder (12, 12', 72, 82) eine Öffnung (72b) aufweist, die mit dem schmalen Schaftabschnitt (72a, 82a) verbunden ist und wenigstens einen Anschlagring (75) beinhaltet, um ein Herunterrutschen des Nadelsitzes (11, 11', 71, 81) zu verhindern und um das Einziehen des Nadelsitzes (11, 11', 71, 81) nach Beendigung der Injektion zu führen.

12. Medizinisches Sicherheitsinjektionsgerät (1) nach Anspruch 1, wobei der Hohlzylinder (12, 12', 72, 82) einen vorderen Teil umfasst, der in der Nähe des Nadelsitzes (11, 11', 71, 81) und einstückig mit einem schmalen Schaftabschnitt (72a, 82a) ausgebildet ist, dessen Durchmesser kleiner ist als der Durchmesser des Hohlzylinders (12, 12', 72, 82), wobei der Hohlzylinder (12, 12', 72, 82) eine Öffnung (72b) aufweist, die mit dem schmalen Schaftabschnitt (72a, 82a) verbunden ist und einstückig mit wenigstens einem Flaschenhalsbereich (85) ausgebildet ist, um ein Herunterrutschen des Nadelsitzes (11, 11', 71, 81) zu verhindern und um das Einziehen des Nadelsitzes (11, 11', 71, 81) nach Beendigung der Injektion zu führen.

## Revendications

1. Injecteur de sécurité médicale (1) comprenant une embase d'aiguille (11, 11', 71, 81) reliée à une aiguille (10, 10' ; 70, 80), un corps cylindrique creux (12, 12', 72, 82) pour retenir l'embase de l'aiguille (11, 11', 71, 81) et, après que l'injecteur de sécurité médicale (1) ait achevé une injection, guider l'embase de l'aiguille (11, 11', 71, 81) pour se rétracter dans le corps cylindrique creux (12, 12', 72, 82) et l'injecteur de sécurité médicale (1) étant **caractérisé en ce que** l'injecteur de sécurité médicale (1) comprend une combinaison de piston (14, 74, 84) disposée à l'intérieur du corps cylindrique creux (12, 12', 72, 82),
la combinaison de piston (14, 74, 84) ayant un piston rétractable (15, 25, 35, 45, 55, 65) et une tige creuse (16, 26, 36, 46, 56),
l'embase de l'aiguille (11) ayant deux extrémités et une extrémité de l'embase de l'aiguille (11) recevant l'aiguille (10) qui s'étend à travers une portion centrale de l'embase de l'aiguille (11), tandis que l'autre extrémité de l'embase de l'aiguille (11) est pourvue d'un bouchon en forme de disque (110) et une pluralité de rainures de positionnement (111) et une pluralité de glissières de rétraction en forme d'U (112) étant reliées aux rainures de positionnement (11) étant prévues à des endroits appropriés entre deux extrémités de l'embase de l'aiguille (11), le corps cylindrique creux (12) ayant une extrémité avant (120) et une extrémité arrière (121) et une pluralité de pièges légèrement élastiques (122) étant prévue à l'extrémité avant (120) et les pièges (122) étant fixés de manière stable par encliquetage dans les rainures de positionnement (111) de l'embase de l'aiguille (11) dans un état non utilisé normal et pendant l'injection, au moins un ressort hélicoïdal rétractable (13) étant prévu pour buter contre le bouchon en forme de disque (110) de l'embase de l'aiguille (11) et étant comprimé à l'intérieur d'un espace du corps cylindrique creux (12, 12', 72, 82) dans un état non utilisé normal et pendant l'injection,
cependant que, lorsqu'une force est appliquée à la combinaison de piston (14, 74, 84) et donc vers l'aiguille (10) après achèvement de l'ïnjection, la saillie qui existe au moins (150, 550, 650) est dégagée de l'évidement qui existe au moins (160), ceci étant dû à la force, et les pièges (122) à l'extrémité avant (120) du corps cylindrique creux (12) sont indirectement forcés à s'étendre de manière élastique et donc de se dégager de l'embase de l'aiguille (11) et le piston rétractable (15, 25, 35, 45, 55, 65) est forcé à glisser dans la tige creuse (16, 26, 36, 46, 56) et l'espace du corps cylindrique creux (12, 12', 72, 82) qui loge le ressort hélicoïdal rétractable comprimé (13) s'étend pour permettre au ressort hélicoïdal rétractable comprimé (13) de se décompresser et de permettre à l'embase de l'aiguille 11, 11', 71, 81) d'être rétractée dans le corps cylindrique creux ('12, 12', 72, 82) dans une direction opposée à une direction de l'injection le long des glissières de rétraction en forme d'U (112), **caractérisé en ce que**
le piston rétractable (15, 25, 35, 45, 55, 65) et la tige creuse (16, 26, 36, 46, 56) sont moulés par injection séparément dans de la matière légèrement élastique et mis ensemble pour permettre à une portion de fond du piston rétractable (15, 25, 35, 45, 55, 65) d'être couplée par un manchon à une portion de dessus de la tige creuse (16, 26, 36, 46, 56) et pour ainsi former une région couplée par un manchon (A) et le piston rétractable (15, 25, 35, 45, 55, 65) a une paroi de côté extérieure formée avec au moins une saillie (150, 550, 650) dans la région couplée par un manchon (A) pour être fixé par encliquetage serré de manière correspondante avec au moins un évidement (160) formé sur une paroi de côté intérieure de la tige creuse (16, 26, 36, 46, 56) dans la région couplée par un manchon (A) dans un état non utilisé normal et pendant l'injection et dans lequel le piston rétractable (15, 25, 35, 45, 55, 65) comprend au moins une découpe d'ajustement de contrainte (151, 551) prévue sur une portion centrale de la région couplée par un manchon (A) ou la tige creuse (16, 26, 36, 46, 56) a un bord (162, 262) pourvu d'au moins une découpe d'ajustement de contrainte (163, 263, 563) dans la région couplée par un manchon (A).

2. Injecteur de sécurité médicale (1) selon la revendication 1, la saillie qui existe au moins (150, 550, 650) étant sélectionnée dans le groupe comportant un endroit en saillie formée en formant une pièce sur le piston rétractable (15, 25, 35, 45, 55, 65), une ou plusieurs rangées de nervures annulaires latérales en saillie (350, 450) adjacentes l'une par rapport à l'autre et une ou plusieurs rangées de nervures annulaires latérales en saillie (350, 450) espacées l'une de l'autre.

3. Injecteur de sécurité médicale (1) selon la revendication 2, l'une quelconque des nervures annulaires latérales en saillie (350, 450) ayant un profil continu ou discontinu.

4. Injecteur de sécurité médicale (1) selon la revendication 1, le piston rétractable (15, 25, 35, 45, 55, 65) ayant un corps de piston dont le bord du fond (152) est arrondi.

5. Injecteur de sécurité médicale (1) selon la revendication 1, l'évidement qui existe au moins (160) étant une rainure et une cavité.

6. Injecteur de sécurité médicale (1) selon la revendication 1, l'évidement qui existe au moins (160) étant sélectionné dans le groupe comportant une ou plusieurs rangées de nervures concaves annulaires (260, 360, 460, 560) adjacentes l'une par rapport à l'autre, une ou plusieurs rangées de nervures de rainures concaves annulaires (260, 360, 460, 560) espacées l'une de l'autre, une ou plusieurs rangées de rainures annulaires (360, 460, 560) adjacentes l'une par rapport à l'autre et une ou plusieurs rangées de rainures annulaires (360, 460, 560) espacées l'une de l'autre.

7. Injecteur de sécurité médicale (1) selon la revendication 6, l'une quelconque des nervures concaves annulaires (260, 360, 460, 560) ou l'une quelconque des rainures annulaires (360, 460, 560) ayant un profil continu.

8. Injecteur de sécurité médicale (1) selon la revendication 6, l'une quelconque, des nervures concaves annulaires (260, 360, 460, 560) ou l'une quelconque des rainures annulaires (360, 460, 560) ayant une section transversale en C ou angulaire circulaire.

9. Injecteur de sécurité médicale (1) selon la revendication 1, la tige creuse (16, 26, 36, 46, 56) ayant une paroi de côté pourvue d'au moins un trou d'aération (264) distal par rapport à la région couplée par un manchon (A).

10. Injecteur de sécurité médicale (1) selon la revendication 1, la découpe d'ajustement de contrainte (151, 551) qui existe au moins étant soit une cavité, soit un trou borgne, soit un trou traversant.

11. Injecteur de sécurité médicale (1) selon la revendication 1, le corps cylindrique creux (12, 12', 72, 82) ayant une section avant qui est près de l'embase de l'aiguille (11, 11', 71, 81) et qui est formée en une pièce avec une portion cylindrique étroite (72a, 82a) qui a un diamètre qui est plus petit qu'un diamètre du corps cylindrique creux (12, 12', 72, 82) et le corps cylindrique creux (12, 12', 72, 82) ayant une ouverture (72b) qui est reliée à la portion cylindrique étroite (72a, 82a) et qui est pourvue d'au moins un bouchon en anneau (75) pour empêcher l'embase de l'aiguille (11, 11', 71, 81) de se rétracter, une fois l'injection achevée.

12. Injecteur de sécurité médicale (1) selon la revendication 1, le corps cylindrique creux (12, 12', 72, 82) ayant une section avant qui est près de l'embase de l'aiguille (11, 11', 71, 81) et qui est formée en une pièce avec une portion cylindrique étroite (72a, 82a) qui a un diamètre qui est plus petit qu'un diamètre du corps cylindrique creux (12, 12', 72, 82) et le corps cylindrique creux (12, 12', 72, 82) ayant une ouverture (72b) qui est reliée à la portion cylindrique étroite (72a, 82a) et qui est formée en une pièce avec au moins une portion de goulot (85) pour empêcher l'embase de l'aiguille (11, 11', 71, 81) de glisser dehors et pour guider l'embase de l'aiguille (11, 11', 71, 81) pour se rétracter, une fois l'injection achevée.
